# EUROPEAN PATENT SPECIFICATION

(11) **EP 2 475 769 B1**
(45) Date of publication and mention of the grant of the patent: **09.11.2016**
(21) Application number: 10813423.0
(22) Date of filing: 06.09.2010
(51) Int. Cl.: C12N 9/10, G01N 33/53, A61K 31/415, A61K 38/45, A61P 35/02, C12Q 1/48, G01N 33/574, G01N 33/68

(54) **INHIBITION OF HISTONE ACETYLTRANSFERASES BY CTK7A AND METHODS THEREOF**
HEMMUNG VON HISTONACETYLTRANSFERASEN DURCH CTK7A UND VERFAHREN DAFÜR
INHIBITION DES HISTONES ACÉTYLTRANSFÉRASES PAR CTK7A ET PROCÉDÉS ASSOCIÉS

(30) Priority: 07.09.2009 IN CH21682009
(43) Date of publication of application: 18.07.2012
(73) Proprietor: Jawaharlal Nehru Centre for Advanced Scientific Research, Bangalore 560 064, Karnataka (IN)
(72) Inventor: KUNDU, Tapas, Kumar, Karnataka 560064 (IN); ARIF, Mohammed, Jharkhand 827012 (IN); MANTELINGU, Kempegowra, Mysore District Karnataka 570001 (IN); SRINIVASACHAR, Gopinath, Kodaganur, Karnataka 560085 (IN)
(74) Representative: Grünecker Patent- und Rechtsanwälte PartG mbB
(86) International application number: PCT/IB2010/053998
(87) International publication number: WO 2011/027330

(56) References cited:
- WO-A2-02/07722
- WO-A2-03/105751
- WO-A2-03/105751
- WO-A2-2005/047457
- WO-A2-2006/004738
- WO-A2-2006/004738
- US-A1- 2006 020 027
- JOONG SUP SHIMA ET AL: "Hydrazinocurcumin, a novel synthetic curcumin derivative, is a potent inhibitor of endothelial cell proliferation", BIOORGANIC & MEDICINAL CHEMISTRY, PERGAMON, GB, vol. 10, no. 9, 1 September 2002 (2002-09-01), pages 2987-2992, XP008154167, ISSN: 0968-0896, DOI: 10.1016/S0968-0896(02)00129-3 [retrieved on 2002-07-03]
- SHIM J S ET AL: "A New Curcumin Derivative, HBC, Interferes with the Cell Cycle Progression of Colon Cancer Cells via Antagonization of the Ca<2+>/Calmodulin Function", CHEMISTRY AND BIOLOGY, CURRENT BIOLOGY, LONDON, GB, vol. 11, no. 10, 1 October 2004 (2004-10-01), pages 1455-1463, XP025940117, ISSN: 1074-5521, DOI: 10.1016/J.CHEMBIOL.2004.08.015 [retrieved on 2004-10-15]
- MARC LACROIX: "Persistent use of "false" cell lines", INTERNATIONAL JOURNAL OF CANCER, vol. 122, no. 1, 1 January 2007 (2007-01-01), pages 1-4, XP055055837, ISSN: 0020-7136, DOI: 10.1002/ijc.23233
- ARIF M ET AL: "Nitric Oxide-Mediated Histone Hyperacetylation in Oral Cancer: Target for a Water-Soluble HAT Inhibitor, CTK7A", CHEMISTRY AND BIOLOGY, CURRENT BIOLOGY, LONDON, GB, vol. 17, no. 8, 27 August 2010 (2010-08-27), pages 903-913, XP027259892, ISSN: 1074-5521 [retrieved on 2010-08-26]
- SHIM, J. S. ET AL.: 'Hydrazinocurcumin, a Novel Synthetic Curcumin Derivative, is a Potent Inhibitor of Endothelial Cell Proliferation' BIOORGANIC & MEDICINAL CHEMISTRY. vol. 10, no. 9, 2002, pages 2987 - 2992, XP008154167
- ERLICH, R. B. ET AL.: 'Valproic acid as a therapeutic agent for head and neck squamous cell carcinomas.' CANCER CHEMOTHERAPY AND PHARMACOLOGY. vol. 63, no. 3, February 2009, pages 381 - 389, XP019655097
- SWAMINATHAN, V. ET AL.: 'Human histone chaperone nucleophosmin enhances acetylation-dependent chromatin transcription.' MOLECULAR AND CELLULAR BIOLOGY. vol. 25, no. 17, 2005, pages 7534 - 7545, XP008154213
- NOTT, A. ET AL.: 'S-Nitrosylation of histone deacetylase 2 induces chromatin remodelling in neurons.' NATURE. vol. 455, no. 7211, 2008, pages 411 - 415, XP008154201
- SEN, N. ET AL.: 'Nitric oxide-induced nuclear GAPDH activates p300/CBP and mediates apoptosis.' NAT CELL BIOL. vol. 10, no. 7, 2008, pages 866 - 873, XP008154204
- ARIF, M. ET AL.: 'Nitric Oxide-Mediated Histone Hyperacetylation in Oral Cancer: Target for a Water-Soluble HAT Inhibitor.' CHEMISTRY & BIOLOGY vol. 17, no. 8, 27 August 2010, pages 903 - 918, XP027259892

## Description

### FIELD OF THE DISCLOSURE

The present disclosure relates to a method for inhibiting histone acetyltransferases by CTK7A. The present disclosure also relates to identification of induction of autoacetylation of p300 and its inhibition by CTK7A.

### BACKGROUND AND PRIOR ART OF THE DISCLOSURE

Oral cancer is one of the most common types of human cancer, with an annual incidence of 274,000 cases world wide¹. Tobacco use and alcohol intake are the major risk factors for the development of oral cancer. However, the exact mechanism by which tobacco carcinogen and alcohol induces transformation and malignant progression of the epithelial cells in oral cancer is not well understood².

The past one decade has seen rapidly increasing evidences suggesting the pivotal role of chromatin structure-function in several disease manifestations^{3,4}. This is evident from the fact that genetic alterations and/or a more diverse group of epigenetic changes may result in disease pathogenesis^{3,4,5-10}. Chromatin being a dynamic entity plays a critical role in all the nuclear related phenomenon like transcription, repair and replication etc¹¹⁻¹². Post translational modifications of chromatin play an important role in maintaining chromatin structure-function and hence regulate gene expression, cell growth and differentiation. It has been suggested that perturbation of the transcriptional state of a cell can lead to developmental defects¹³. Further it has also been shown that dysfunction of different chromatin components and covalent modifications of histones can lead to disease pathogenesis^{3,4,13,14}.

Reversible histone acetylation is one of the well characterized epigenetic modifications and is catalyzed by histone acetyltrasnferases (HATs) and histone deacetylases (HDACs), which affect the acetylation of histone and non-histone proteins thereby playing significant role in the down stream biological functions^{16,17}. Altered HAT and HDAC activity are now known to play important role in several diseases including cancer^{7-9,15,18,19}. The histone acetyltransferase, p300 is a global transcriptional coactivator and is a major HAT in the cell²⁰ High levels of p300 has been observed in some tumors^{21,22}. Further, mutations in p300 acetyltransferase were found in primary tumors and cell lines²³. Similarly, loss of heterozygocity at the p300 locus is associated with the colorectal, breast cancer and with brain cancer (gliobastoma)^{19,23}. Although these data indicate the involvement of CBP, p300 and PCAF genes, HAT activities of these acetyltransferases have not been established as the cause of the malignancy¹⁹.

Recently, alteration of histone modifications in different cancers have been reported.

The loss of Lys 16 acetylation and Lys 20 methylation of H4 are found to be associated with primary tumors and tumor cell lines⁵. In another study, changes in bulk histone modifications of cancer cells were found to be predictive of clinical out come in prostate cancer⁷. However, with a rare exception, hyperacetylation of histones has been observed in hepatocellular carcinoma²⁴. Apart from cancer, dysfunction of lysine acetyltransferases have been implicated in other diseases: inflammatory processes, Huntington disease, cardiac disease, diabetes and AIDS²⁵⁻²⁸. These observations suggest that specific and relatively non-toxic inhibitors of acetyltransferases could be considered as new generation therapeutic agents, specially, for cancer. Recently, several HAT inhibitors have been discovered^{15,29}, which have been shown to possess, potential clinical impact in cancer, HIV and cardiac disease^{26,30-32}. However, effect of HAT inhibitor in cancer manifestation has not been tested yet.

US2006/020027 discloses the use of curcumin derivatives to inhibit HATs, including p300/CBP or PCAF. This inhibition is discussed in relation to diseases where HATs are known to be involved, e.g. cancer.

Joong Sup Shima et al. (2004) disclose curcumin derivatives, including compound HBC, and their anti-angiogenic properties, and thus as a way to inhibit tumor growth and metastasis.

Cancer is marked by hyperproliferative cells which have evaded the apoptotic machinery of the cells and hence have overexpression of antiapoptotic proteins. NPM1 (also known as B23)³³ and GAPDH^{34,35} are two of those genes which are known to get frequently up-regulated in many cancers. Both of these proteins are suggested to be positive regulators of cell proliferation.

### STATEMENT OF THE DISCLOSURE

Accordingly, the present disclosure relates to:
1) An in vitro method of inhibiting histone acetyltransferases (HATs) by Sodium-4-(3,5-bis(4-hydroxy-3-methoxystyryl)-1H-pyrazol-1-yl)benzoate (CTK7A), said method comprising step of incubating the HATs with CTK7A, wherein the HATs are CREB binding protein (p300/CBP) and P300/CBP Associated factor (PCAF).
2) The method of claim 1, wherein HAT inhibitory concentration of the CTK7A ranges from 25pM to 200 µM I preferably 40 µM to 80 µM.
3) A therapeutically acceptable amount of Sodium-4-(3,5-bis(4-hydroxy-3-methoxystyryl)-1H-pyrazol-1-yl)benzoate (CTK7A) for use in treating cancer, optionally along with pharmaceutically acceptable excipients, wherein the CTK7A inhibits acetyltransferase activity of HATs and thereby inhibits hyperacetylation of histones.
4) The therapeutically acceptable amount of CTK7A for use according to 3, wherein CTK7A is to be administered intraperitonially.
5) The therapeutically acceptable amount of CTK7A for use according to 3, wherein the CTK7A reduces tumor size of the cancer by 50%.
6) The therapeutically acceptable amount of CTK7A for use according to 3, wherein the CTK7A induces polyploidy in cancer cells to induce senescence like growth arrest.
7) The therapeutically acceptable amount of CTK7A for use according to 3, wherein the cancer is oral squamous cell carcinoma.
8) The therapeutically acceptable amount of CTK7A for use according to 3, wherein the CTK7A is further administered along with an epigenetic drug target molecule or pharmaceutically acceptable chemotherapeutic or a combination thereof.
9) An in vitro method of inhibiting autoacetylation of p300 by Sodium-4-(3,5-bis(4-hydroxy-3-methoxystyryl)-1H-pyrazol-1-yl)benzoate (CTK7A), said method comprising steps of: reacting and incubating full length radio-labeled p300 with predetermined concentrations of CTK7A, optionally along with cocktail of HDAC inhibitors, and performing filter binding assay and identifying inhibition of autoacetylation of p300 by fluorography and autoradiography.
10) Sodium-4-(3,5-bis(4-hydroxy-3-methoxystyryl)-1H-pyrazol-1-yl)benzoate (CTK7A).
11) A process for preparing Sodium-4-(3,5-bis(4-hydroxy-3-methoxystyryl)-1H-pyrazol-1-yl)benzoate (CTK7A), said process comprising the step of reacting hydrazinobenzoylcurcumin with sodium ethoxide to obtain Sodium-4-(3,5-bis(4-hydroxy-3-methoxystyryl)-1H-pyrazol-1-yl)benzoate (CTK7A).

### BRIEF DESCRIPTION OF THE ACCOMPANYING FIGURES

**Figure 1****:** Shows histones are hyperacetylated in KB cells.
**Figure 2a****:** Shows Immunohistochemical detection of histone acetylation and expression of different proteins in oral cancer samples.
**Figure 2b****:** Shows western blotting analysis to compare the protein levels and acetylation status of histone H3 in tumor and respective adjacent normal tissue of the different patient samples *(left panel*) and Quantification of respective bands (*right panel*)
**Figure 3a****:** Shows immunohistochemical detection of iNOS and COX-2 expression in human oral cancer.
**Figure 3b****:** Shows GSNO induce expression of NPM1 and GAPDH. Cells were treated with indicated concentration of GSNO for 24 h.
**Figure 3c****:** Shows that KB cells were grown in presence of 200 µM GSNO or GSH for 24 h. Cell lysates were immunoprecipitated with anti-acetyl lysine antibody and the immunoprecipitate were analysed by western blotting with an anti- NPM1 (*left panel*) and anti-GAPDH (*right panel*) antibodies.
**Figure 3d****:** Shows IFNγ treatment enhances acetylation of NPM1 and GAPDH which is abolished by the iNOS inhibiotor 1400W (100 µM).
**Figure 3e****:** Shows GAPDH translocates to the nucleus of KB cells after exposure to IFNγ. KB cells were treated with IFNγ (10 ng ml⁻¹) for 16h and stained with anti-GAPDH antibodies (green) and DAPI (blue).
**Figure 3f****:** *Left panel* shows autoacetylation of p300fl was examined with ³H-acetyl CoA in presence or absence of NPM1. GAPDH (600 ng) was used as a positive control. *Right panel* shows NPM1 enhances the p300 autoacetylation in concentration dependent manner.
**Figure 3g****:** Shows that NO caused the hyperacetylation of Histone H3K14. Histones were isolated from the GSNO treated KB cells and subjected to western blotting with anti-acetylated H3K14 antibody.
**Figure 4a****:** Shows structural formula of CTK7A
**Figure 4b****:** Shows Inhibition curves for various recombinant HATs, HMTs and HDACs.
**Figure 4c****:** Shows that CTK7A is a non-competitive inhibitor of p300. Lineweaver-Burk plots for effect of CTK7A on p300 mediated acetylation of highly perified HeLa core histones. Filter binding assays were performed in the presence and absence of 30 and 50 µM CTK7A with different substrate concentrations.
**Figure 4d (A)**: Shows CTK7A inhibits autoacetylation of full length p300 *in vitro*. Autoacetylation assays were performed using p300fl in the absence and in the presence of ³H-acetyl CoA with indicated concentration of CTK7A. Figure 4g:

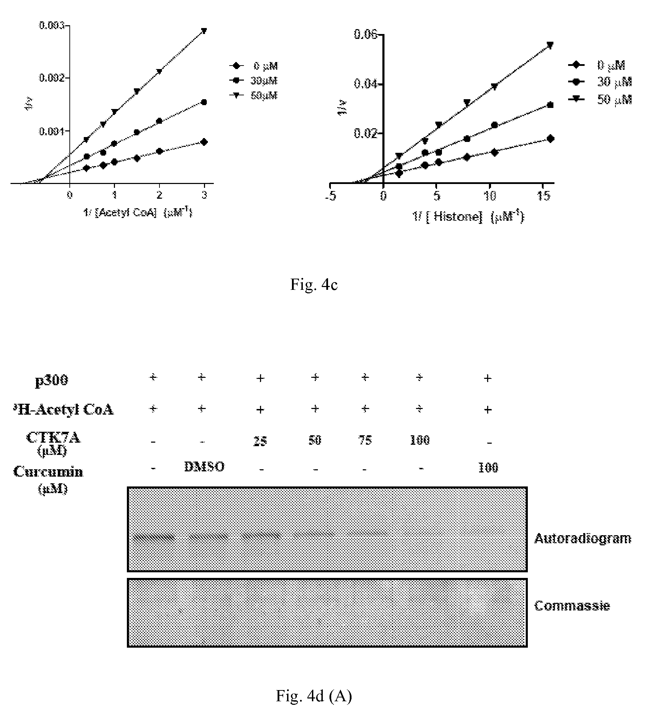

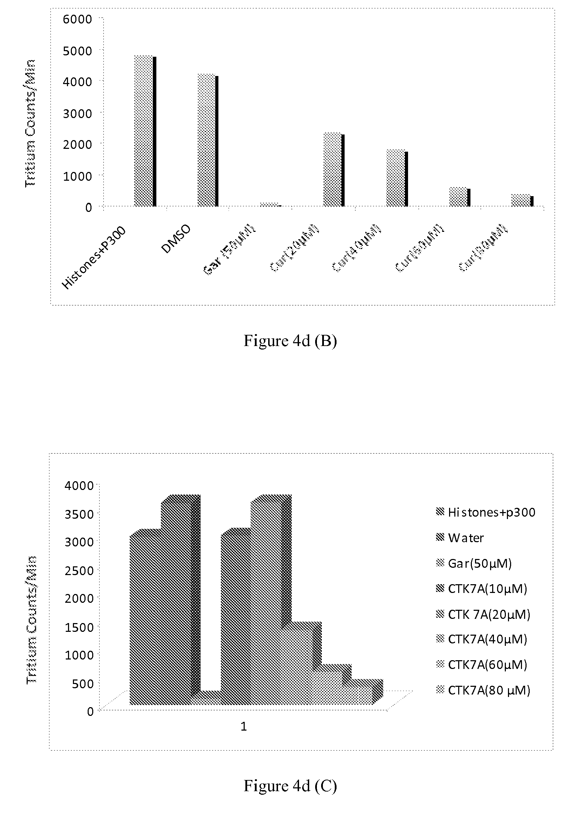

**Figure 4d (B):** Shows the inhibitory effect of curcumin on p300 activity by Filter binding histone acetyltransferase (HAT) assay.
**Figure 4d (C):** Shows the inhibitory effect of CTK7A on p300 activity by Filter binding histone acetyltransferase (HAT) assay.

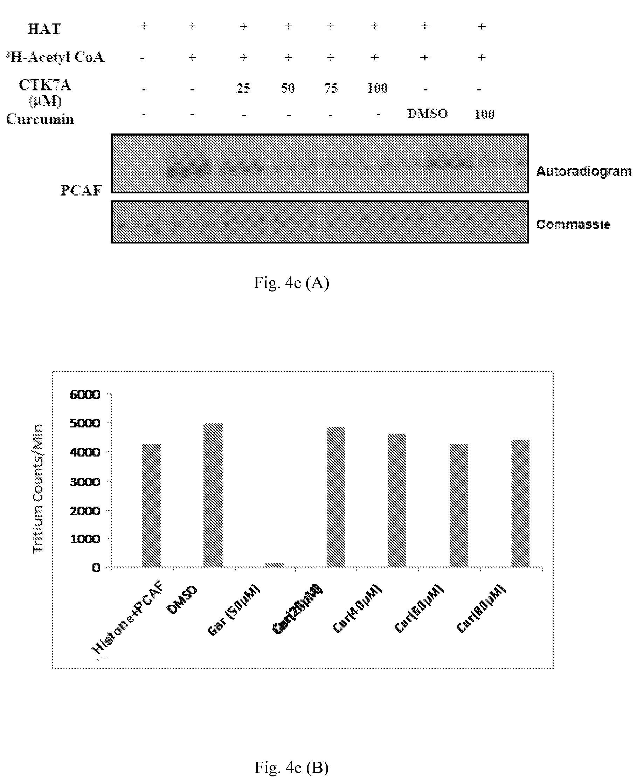

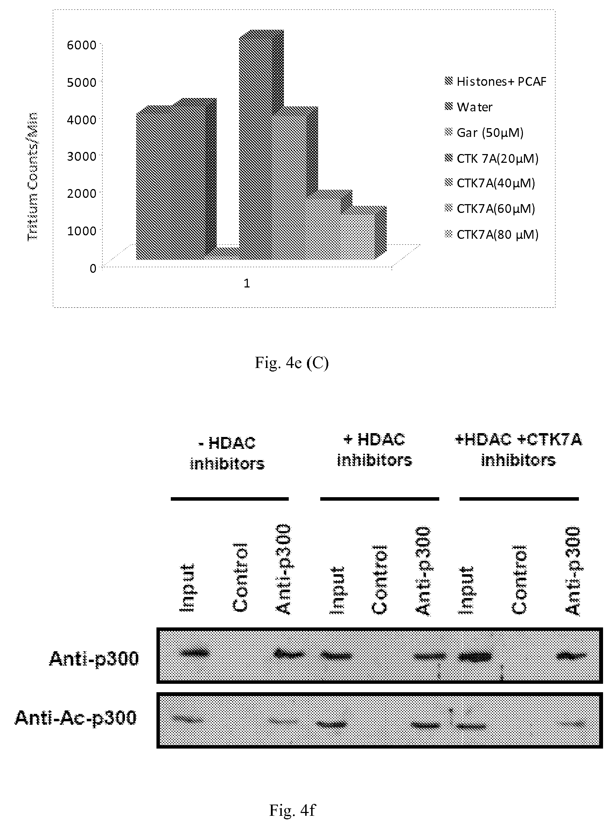

**Figure 4e (A):** Shows CTK7A inhibits PCAF autoacetylation *in vitro*.
**Figure 4e (B):** Shows the inhibitory effect of curcumin on PCAF activity by Filter binding histone acetyltransferase (HAT) assay.
**Figure 4e (C):** Shows the inhibitory effect of CTK7A on PCAF activity by Filter binding histone acetyltransferase (HAT) assay.
**Figure 4f****:** Shows CTK7A inhibits p300 autoacetylation *in vivo*

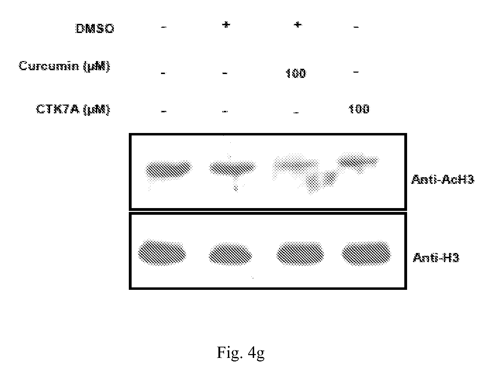

**Figure 4g****:** Shows CTK7A inhibits histone acetylation in KB cells.

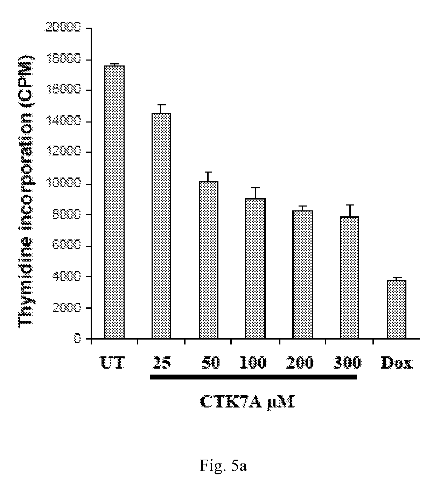

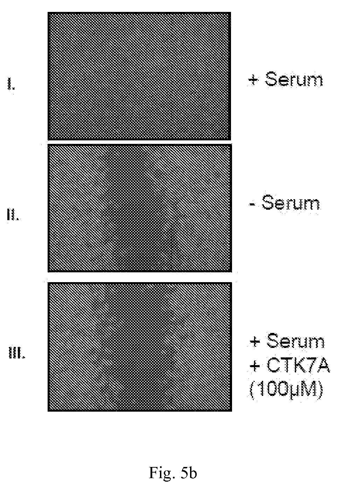

**Figure 5a****:** Shows CTK7A inhibits the growth of KB cells.
**Figure 5b:** Shows CTK7A inhibits wound healing.

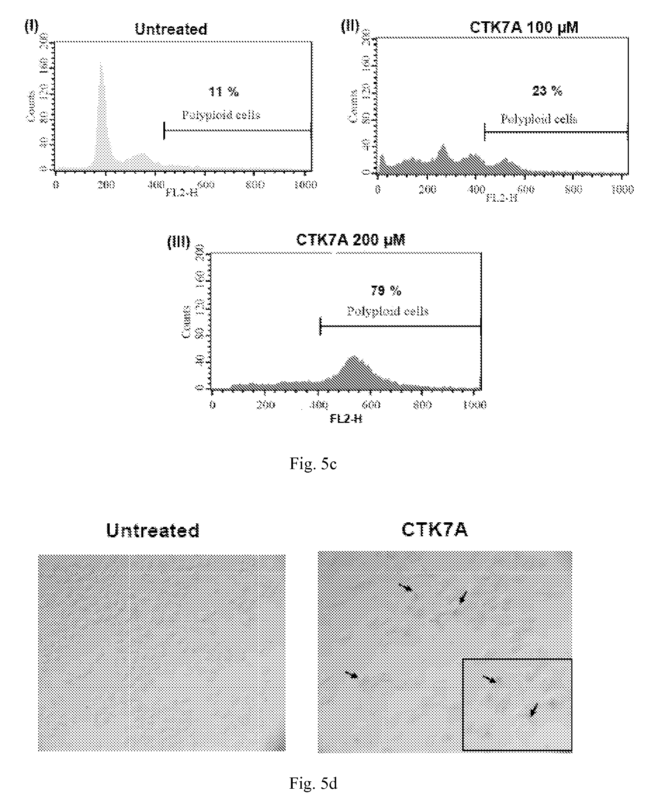

**Figure 5c****:** Shows CTK7A induces polyploidy in KB cells.
**Figure 5d****:** Shows CTK7A induces Senescence-associated β-gal expression (SA-β-gal) in KB cells.

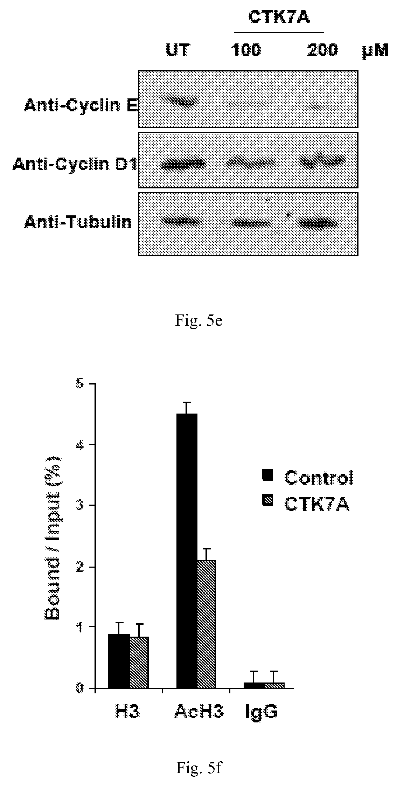

**Figure 5e****:** Shows CTK7A affects cell cycle progression by inhibiting cyclinE expression.
**Figure 5f****:** Shows CTK7A inhibits the H3 acetylation at cyclinE promoter.

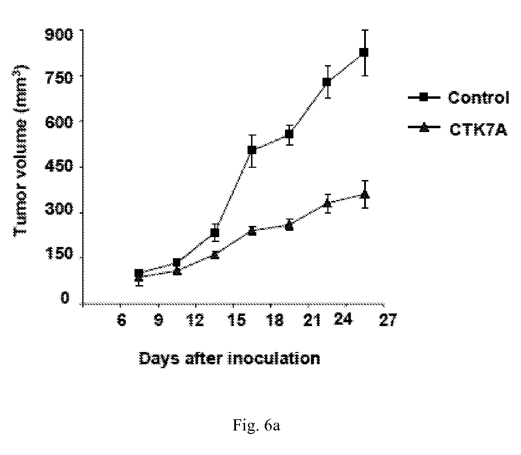

**Figure 6a****:** Shows the nude mice carrying the KB cell xenografts were treated with phosphate buffered saline (control) or with CTK7A intraperitonially with 100 mg/Kg body weight/ twice a day. One-way ANOVA revealed that tumor sizes were significantly different (p < 0.05).

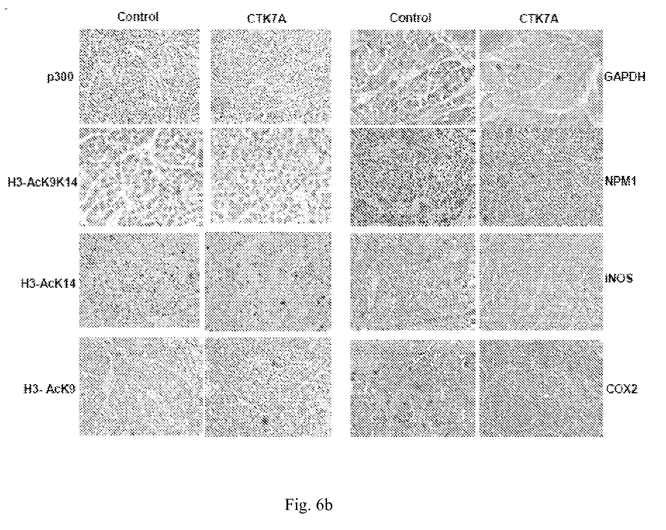

**Figure 6b****:** Shows CTK7A inhibits histone acetylation in nude mice. KB cell tumors from control and CTK7A treated mice were used for immunohistochemical detection with indicated antibodies. Arrow mark in GAPDH IHC indicates the nuclear localization of GAPDH while it is absent in CTK7A treated tumor.

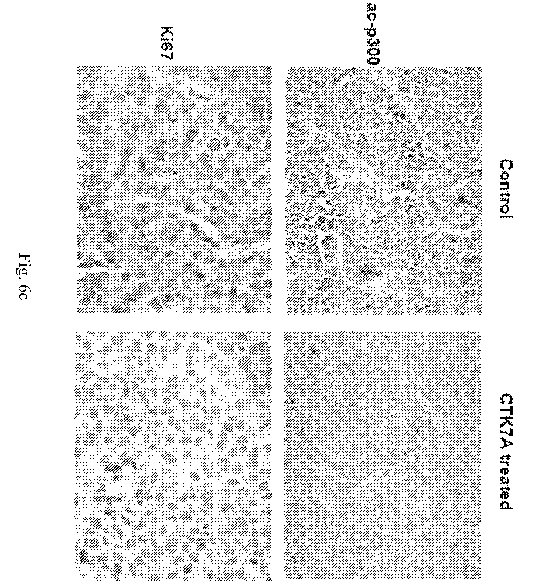

**Figure 6c****:** Shows that KB cell tumors from control and CTK7A treated mice were used for immunohistochemical detection with indicated antibody

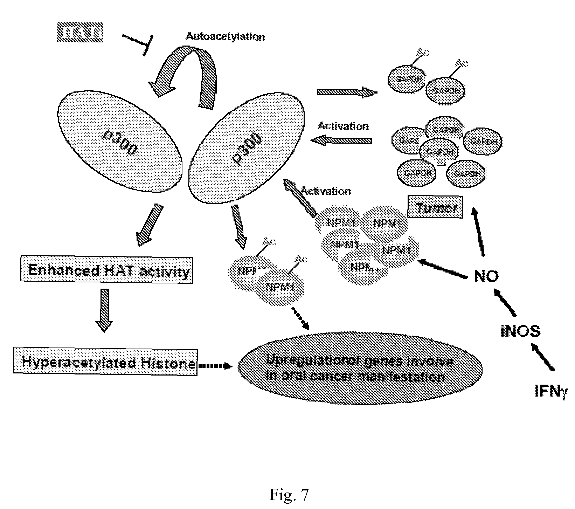

**Figure 7****:** Shows Putative epigenetic signaling pathway which cause hyperacetylation of histone and nonhistone proteins.

### DETAILED DESCRIPTION OF THE DISCLOSURE

The present disclosure is related to a method of inhibiting histone acetyltransferase (HAT) by Sodium-4-(3,5-bis(4-hydroxy-3-methoxystyryl)-1*H*-pyrazol-1-yl)benzoate (CTK7A), said method comprising step of incubating the HAT with CTK7A.

In said method HAT is selected from a group comprising p300/CBP (CREB binding protein) and PCAF (P300/CBP Associated factor) In yet another embodiment, in the said method HAT inhibitory concentration of the CTK7A ranges from about 25µM to about 200 µM, preferably about 40 µM to about 80 µM.

The present disclosure is also related to CTK7A for use in a method of treating cancer, comprising administering therapeutically acceptable amount of CTK7A, optionally along with pharmaceutically acceptable excipients to a subject in need thereof.

In another embodiment the CTK7A inhibits acetyltransferase activity of HATs and thereby inhibits hyperacetylation of histones.

In yet another embodiment, the route of administration is intraperitonial.

In still another embodiment, the CTK7A reduces tumor size of the cancer by about 50%.

In still another embodiment, the CTK7A induces polyploidy in cancer cells to induce senescence like growth arrest.

In still another embodiment, the cancer is oral squamous cell carcinoma.

In still another embodiment, the CTK7A is further administered along with an epigenetic drug target molecule or pharmaceutically acceptable chemotherapeutic or a combination thereof.

The present disclosure is also related to an in vitro method of inhibiting autoacetylation of p300 by Sodium-4-(3,5-bis(4-hydroxy-3-methoxystyryl)-1*H*-pyrazol-1-yl)benzoate (CTK7A), said method comprising steps of:
a. reacting and incubating full length radio-labeled p300 with predetermined concentrations of CTK7A, optionally along with cocktail of HDAC inhibitors, and
b. performing filter binding assay and identifying inhibition of autoacetylation of p300 by fluorography and autoradiography.

Altered histone acetylation pattern is associated with several diseases including cancer. Dysfunction of histone deacetylases (HDACs) and the consequent hypoacetylation of histone as well as nonhistone proteins have been causally related to cancer manifestation. Unlike most of the cancers, the present disclosure reports that histones are found to be highly hyperacetylated in oral cancer patient samples. Mechanistically, overexperssion as well as enhanced autoacetylation of p300 induced by NPM1 and GAPDH causes the hyperacetylation, which is nitric oxide (NO) signal dependent. Inhibition of the acetyltransferase activity (HAT) of p300 by a newly synthesized, water soluble, small molecule inhibitor could substantially retards/inhibits the xenografted oral tumor growth in mice. These results, therefore, not only establish a new epigenetic target for oral cancer but also put forward a HAT inhibitor as potential therapeutic molecule.

The present disclosure shows that histone (H3) is hyperacetylated in oral cancer patient samples and is positively correlated to the upregulated NPM1 and GAPDH protein levels. The disclosure also presents a mechanism to explain how hyperacetylation of H3 could be regulated by NPM1 and GAPDH in a nitric oxide (NO) dependent manner involving p300 acetyltransferase. Furthermore a water soluble HAT inhibitor, CTK7A has been shown to inhibit oral tumor cell growth in nude mice.

### EXPERIMENTAL PROCEDURES

### Histone isolation from HeLa nuclear pellet:

Core histones were purified from HeLa nuclear pellet as described elsewhere³¹.

### Purification of histone-modifying enzymes from baculovirus-infected Sf21 cells:

Recombinant baculovirus expressing full-length FLAG-tagged CARM1,CBP and PCAF were purified by immunoaffinity chromatography using M2-agarose beads followed by elution with FLAG peptide. Baculovirus-expressed full-length hexahistidine-tagged p300, and G9a were purified using Ni-NTA affinity chromatography as described previously³⁰.

### HAT Assay:

HAT gel fluorography/autoradiography assays were performed as previously reported³⁰. Kinetic analysis of p300 HAT inhibition was performed as reported earlier³¹ in the presence of (0, 30 and 50 µM) CTK7A. The obtained values were plotted as a lineweaver burk plot using Graphpad Prism software. For p300 Autoacetylation assay, reactions of p300 full length (80ng) were carried out in HAT assay buffer at 30 °C for 10 minutes with or without the protein (NPM1) followed by addition of 1 µl of 4.7 Ci/mmol [³H] acetyl CoA (NEN-PerkinElmer) and were further incubated for another 10 min in a 30 µl reaction. GAPDH was used as positive control. The radio-labeled acetylated p300 were processed by fluorography followed by autoradiography.

### Histone Deacetylase assay (HDAC Assay) and Histone methyltransferase (HMTase) assay:

The deacetylation assay was performed as per standard protocol³¹. For SirT2 deacetylase assay, 50ng of bacterially expressed, recombinant enzyme was added in the presence or absence of cofactor NAD⁺. HMTase was performed as reported earlier³¹.

### Cell culture and Whole cell extract preparation:

KB cells were maintained in Dullbecco's modified Eagle medium (DMEM) with 10% fetal bovine serum (FBS) at 37 °C with a 5% CO2 atmosphere in a humidified incubator. For IFNγ and GSNO treatment, KB cells were incubated in the presence of IFNγ 10 ng ml⁻¹ for an additional 16 h and 24 h respectively. Immunofluorescent staining of cells for confocal microscopy was carried out as described previously³⁰. CTK7A treatment was done for 24 h followed by acid extraction of histone and immunoblotting as per standard protocol³¹. For whole cell extract preparation RIPA buffer (Tris-HCl 50 mM, pH 7.4 NP-40 1%, Na-dcoxycholate 0.25%, NaCl 150 mM, EDTA 1 mM, Phenylmethylsulfonyl fluoride (PMSF), 1 mM Na₃VO₄, 1 mM NaF and protease inhibitor cocktail) was used.

### Immunoprecipitation assay:

Whole cell extract were prepared using RIPA buffer as mentioned above. The pre-blocked protein-G sepharose-bound antibody (4µg) was incubated with 500 µg whole cell lysate overnight at 4°C. After extensive washes, bead-bound protein were analysed by western blotting with indicated antibodies. For HDAC inhibitors treatment, 5 mM sodium butyrate, 5 mM nicotiamide and 100 ng ml⁻¹ TSA were added 10 h before cells were harvest³⁶.

### Chromatin immmunoprecipitation assay (ChIP):

The pull-downs for ChIP assay was performed using anti-acetylated H3 (H3AcK9AcK14) (Santa Cruz) antibody. KB cells were maintained in DMEM supplemented with 10% FBS were treated with CTK7A and cells were grown for 24 hrs. For ChIP assay cells were processed as described elsewhere⁴⁷. The pulldown was done using antibody against the above antibody and the immunoprecipitated samples were deproteinized and ethanol-precipitated to recover the DNA. Real-time PCR analysis was performed using primers for the cyclin E promoter region. PCR primers for the cyclin E promoter region were 5'-GGCGGGACGGGCTCTGGG-3' and 5'-CCTCGGCATGATGGGGCTG-3'.

### Immunohistochemistry:

After deparafinizing, slides were rinsed in ethanol. Antigen retrieval was performed with sodium citrate (10 mM pH 6.0) for 5 min at 98 °C . The staining was performed with the Envision kit (Dako, Denmark). Counterstaining was performed with Mayer's haematoxylin, and mounted in DPX and air dried. Each of the sample specimens was pathologically confirmed before carrying out the immunohistochemistry. For statistical analysis, cells (100 cells from a total of 5-6 independent fields) were counted from both normal and tumor tissue sections and scored for percentage of positive cells (brown colour) for the indicated antibodies. For comparison one-way ANOVA was performed using "Sigmaplot Software". P- value < 0.01 was considered as statistically significant, n= 31.

### Xenograft growth assay:

Animal experiments were performed with the approval by authorized ethics Committee. Sixteen nude mice (BALB/c) were included in the experiments (10 male and 6 female mice). Mice were kept in isolators in a pathogen free environment. All nude mice were 3-4 weeks old at the start of the experiments. KB cells, 2 x 10⁶ cells were inoculated in each mice in the right and left flanks, respectively. Mice were then divided in two groups (5 males and 3 females) in each group. After the tumors grown to palpable in size, CTK7A treatment were given intraperitoneally (i.p) with 100mg/kg body weight/twice a day. Tumors were measured once in three days with a caliper and their volumes were calculated by the formula: 0.52 x D1 x (D2)², where 'D1' and 'D2' are, respectively, the longest and shortest dimension. The last dose of the compound (CTK7A) was administered four hour before sacrificing it. Tumors were removed and put into liquid nitrogen or were fixed in 10% formalin for 1-2 h to make blocks to be used for IHC. For comparing tumor sizes between treated and control one-way ANOVA was performed using "Sigmaplot Software". P- value < 0.05 was considered as statistically significant.

### Fluorescent Activated Cell Sorting (FACS):

KB cells were grown in the presence or absence of CTK7A for 24 h with the indicated concentration. Cells were grown in presence of CTK7A for 2 h in absence of serum followed by addition of 10% Serum. Briefly cells were harvested by mild trypsinization (0.25%) followed by centrifugation at 2000 rpm for 10 minutes at 4°C. Cells were washed with cold PBS by centrifugation at 2000 rpm for 10 minutes at 4°C. Cells were fixed in cold 70% Ethanol which was added drop wise along with mild vortexing.

Samples were left for 12 hrs, after which Ethanol was removed followed by two washes in cold PBS. RNase (100 µg/ml) treatment was subsequently given at 37°C/30 minutes to ensure only DNA staining. 50 µg/ml Propidium Iodide was added for staining. Cells were sorted and analyzed by flow cytometry for the cell cycle distribution using inbuilt software of BD FACScalibur instrument. Analysis was done in FL2 channel.

### In vitro Wound-Healing assay:

Cells in medium containing 10% FBS were seeded in 30 mm dishes. After the cells grew to confluence, wounds of constant diameter were made by sterile plastic pipette tip (200-µl) by scratching the monolayers. Cells were washed with the serum free medium twice and refreshed with medium with or without 10% FBS. Cells were treated with or without the HAT inhibitor for 24 h along with 10% serum. The wound photographs were taken under phase-contrast microscope. Serum positive and negative cells act as positive and negative control for the experiments respectively.

### Senescence-associated β-gal (SA-β-gal) activity analysis:

SA-β-gal activity was analyzed in KB cells as described earlier⁴⁷.

### [³H]Thymidine Incorporation Assay:

Cells were grown in 24-well plate and were treated with the compound for 16 h followed by addition of 1 micro curie [³H] thymidine (NEN, Perkin Elmer). Cells were further grown for an additional period of 8 h. Following this media was aspirated and cells were washed with 1 ml ice cold PBS. Cells were lysed by repeated freeze thawing (2 times). DNA was isolated using cell harvester and scintillation counting was done using liquid scintillation counter.

### General procedure for the synthesis of CTK7A:

### The procedure involves the following two steps:

### A. Preparation of Hydrazinobenzoylcurcumin (CTK7):(insoluble in water)

To a solution of curcumin in methanol (10 mg, 0.027 Mmol) 4-hydrazinobenzoic of acetic acid (2 ml) was added. After incubation for 24 h, the solvent was evaporated in vacuum. The residue acid (20 mg, 0.135 mmol), triethylamine (18.8 µL, 0.135 mmol), and catalytic amount was purified with repeated recrystallization and filtration method using TLC (CHCl₃/MeOH=4:1; *R_{f}*=0.5). This gave CTK7 as a dark orange powder (7.18 mg, 55%) which was analyzed by H¹ NMR, Melting point test, Solubility test and EST-MS.

### B. Preparation of Sodium-4-(3,5-bis(4-hydroxy-3-methoxystyryl)-1H-pyrazol-1-yl)benzoate (CTK7A): (soluble in water)

To a solution of CTK7 in methanol (50mg, 0.1031mmol), (10 ml) of Sodium ethoxide was added (57mg, 0.1175mmol) and the reaction mixture was stirred at room temperature for 90 minutes. The solvent was evaporated in vacuum and the residue was washed with hexane, diethyl ether and ethyl acetate. Product was confirmed through H¹ NMR, Solubility test, melting point.

The present disclosure is further elaborated with the help of following examples and accompanying figures. However, these examples should not be construed to limit the scope of the disclosure.

### Example 1

### Hyperacetylation of histone at H3K14 is linked to the overexpression of NPM1 and GAPDH in oral cancer:

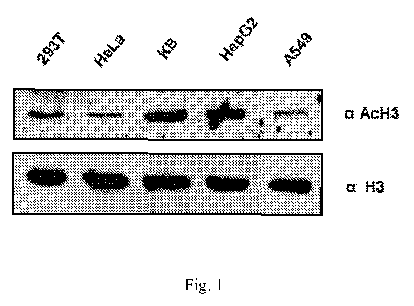

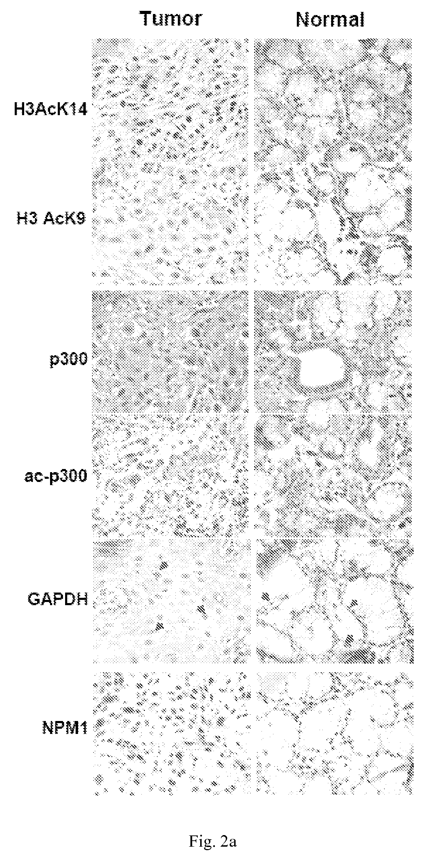

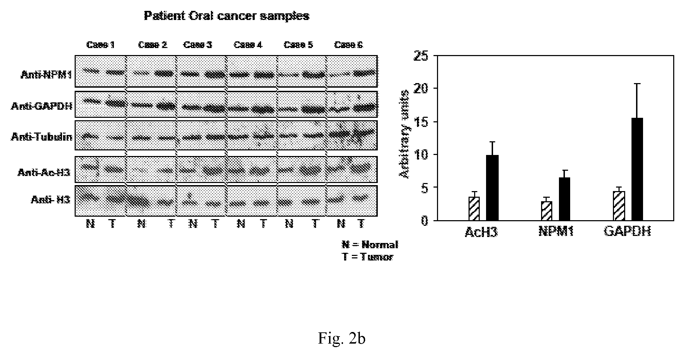

To investigate the status of histone acetylation in different cancers, initially histones were isolated from different cell lines and subjected to immunoblotting analyses with anti-acetylated histone H3 (anti-H3AcK9AcK14) antibodies. It was observed that histones are predominatly hyperacetylated in oral (KB) and the liver (HepG2) cancer cell lines **(****Fig.1****).** Histones were isolated from different cell lines cells as indicated and histone acetylation was analysed by western blotting with anti-acetylated H3 (anti-H3AcK9AcK14) antibody. Anti-H3 was used as a loading control. Although hyperacetylation of histones in hepatocarcinoma has been recently reported²⁴, for oral cancer cell line: almost equivalent enhanced acetylation of histone H3 was quite interesting. These results led us to find out the acetylation levels of histone H3 in the tissues from oral cancer patient samples. By employing, immunohistochemistry (IHC) **(****Fig. 2a****)** and western blotting **(****Fig. 2b****)** analyses using specific antibodies (anti-H3AcK14 and anti-H3AcK9), it was found that histone (predominantly H3K14) are hyperacetylated in the cancerous tissues in comparison to normal tissue **(****Fig. 2a****).**

As H3K14 is the predominant in vivo target of p300 mediated acetylation, the expression levels of p300 was investigated. It was found that p300 significantly overexpressed in the malignant tumor part as compared to the adjacent normal tissue **(****Fig. 2a****).** Since, autoacetylation of p300 enhances its acetyltransferase activity³⁶, the autoacetylation status of p300 was also verified, using a polyclonal antibody, which specifically recognize the acetylated-p300 (ac-p300) molecules³⁶. Interestingly, p300 was found to be hyperacetylated in oral cancer samples **(****Fig. 2a****).** These results suggest that highly active acetylated-p300 could be involved in the histone hyperacetylation at H3K14 in malignant oral tumor.

Autoacetylation of p300 could be enhanced by several factors³⁶⁻⁴⁰ and some of which overexpress in different cancers. In this context, it was found that in the oral tumor tissues, a significant increase in the GAPDH and NPM1 protein levels as compared to corresponding normal tissue in each case (patient samples) **(****Fig. 2a****).** Subsequently, six different pairs of tissue samples were taken (tumor and corresponding adjacent normal tissue) and the levels of protein overexpression were determined by western blotting analysis. It was observed that in all the six pairs of tissue samples histones H3 were hyperacetylated as probed by anti-H3AcK9AcK14 acetylation specific antibody **(****Fig. 2b****).** Both GAPDH and NPM1 were found to be overexpressed in all tumor tissue samples **(****Fig. 2b****).** It was noticed that H3 hyperacetylation follows the same pattern of NPM1 and GAPDH overexpression in all the cancerous tissue samples analyzed. Taken together, these data suggest that overexpression of GAPDH and NPM1 are positively correlated to histone hyperacetylation in oral cancer. An interesting question raised at this juncture is the possibility of this being related to the autoacetylation of p300.

### Example 2

### NO induced H3K14 acetylation is associated with NPM1 and GAPDH overexpression via p300 autoacetylation:

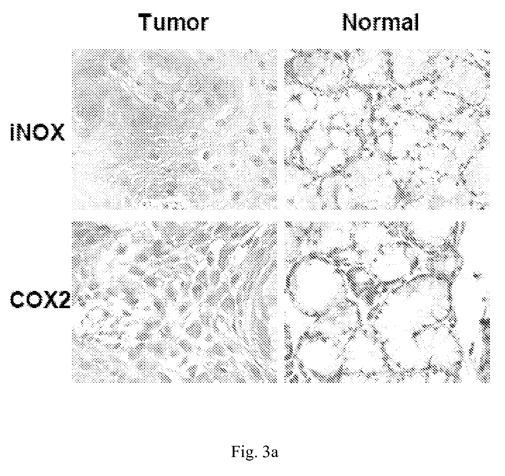

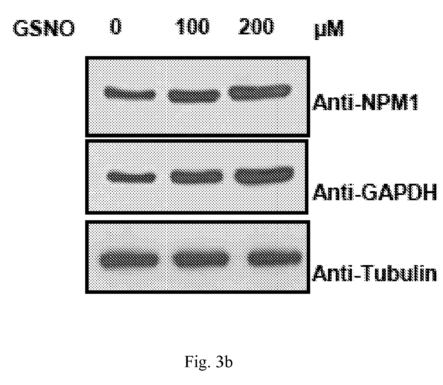

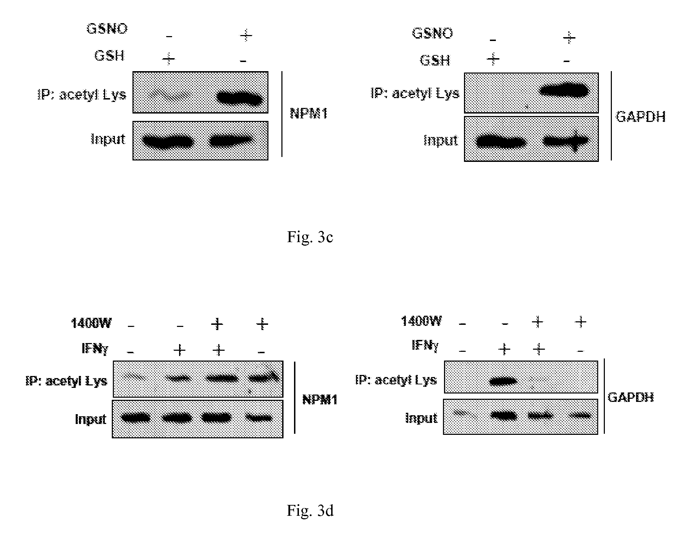

The free radical gas, NO, is generated by nitric oxide synthase (NOS) family of enzymes. NO is a plieotropic signaling molecule that has been identified as mediator for numerous physiological and pathophysiological conditions⁴¹. Since increased production of NO was noticed in oral cancer with a simultaneous upregulation of inflammatory (predominantly NFk-B responsive) genes^{42,43}, it was hypothesized that NO signaling could be associated with autoacetylation of p300, overexpression of GAPDH and NPM1 and hyperacetylation of histones. It was observed that indeed the iNOS levels are significantly enhanced in tumor tissue samples **(****Fig. 3a****).** COX2 levels were also found to be higher in these tumor tissue samples **(****Fig. 2a****).** Recent report suggest that NO dependent and nuclear localized GAPDH enhance p300 autoacetylation and thereby its catalytic activity³⁷. it was found that GAPDH is predominantly localized in the nucleus of oral cancer patient samples **(****Fig. 2a****, as indicated by arrow).** Further, it was observed that when the KB cells were treated with the NO donor, S-nitroso-glutathione (GSNO), the expression of both NPM1 and GAPDH enhanced in a concentration dependent manner **(****Fig. 3b****).** In agreement with the previous report³⁷, it was also found that GAPDH gets acetylated in a NO dependent manner **(****Fig. 3c****).** These results led us to determine the role of NO on NPM1 acetylation. Interestingly, following GSNO treatment it was detected that the acetylation of NPM1 is dramatically enhanced in KB cells **(****Fig. 3c****).**

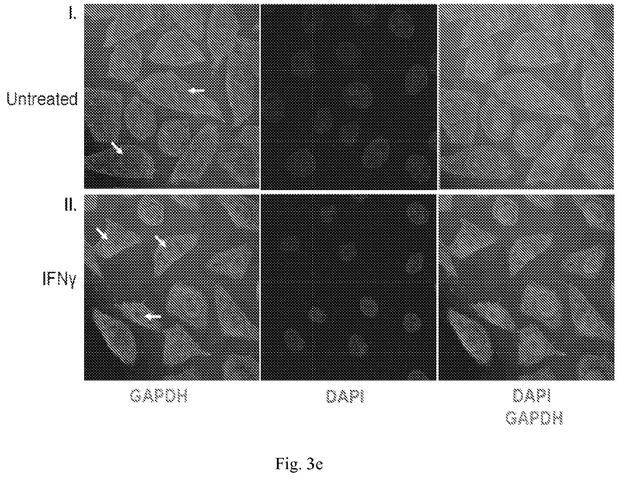

In order to get an insight into the signaling pathways, the affect of IFNγ on GAPDH and NPM1 acetylation in KB cells was investigated, as it is known to activate iNOS gene expression to produce NO⁴⁴. It was found that IFNγ efficiently, enhanced the GAPDH and NPM1 acetylation in NO-dependent manner in KB cells **(****Fig. 3d****),** which was abolished or reduced by the treatment with a specific iNOS inhibitor, N-(3-(Aminomethyl)benzyl)acetamidine (1400W). Furthermore, it was found that IFNγ treatment could induce the translocation of the cytosolic protein, GAPDH to nucleus **(****Fig. 3e****).** Taken together, these results suggest the involvement of NO- signaling in the overexpression of NPM1 and GAPDH and their acetylation in KB cells, which significantly correlates with the observation that in oral cancer tissue samples NPM1, GAPDH and iNOS are overexpressed (as mentioned above).

In the tumor tissue, histone H3K14 was also found to be hyperacetylated **(****Fig. 2a****).**

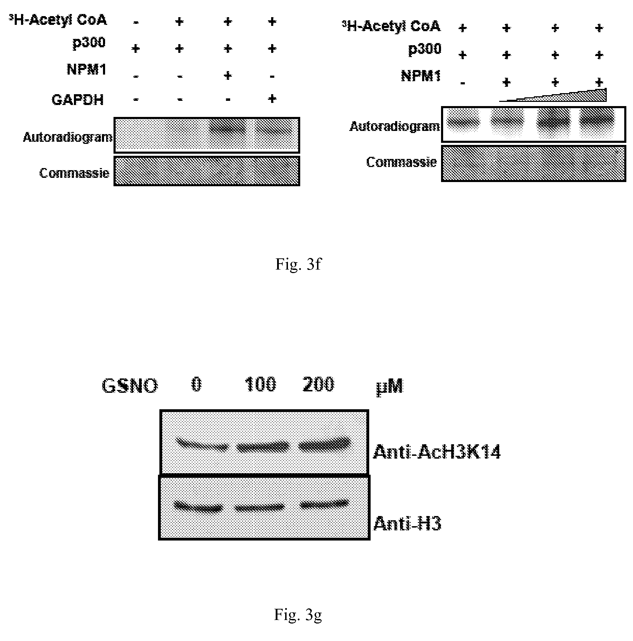

These observations prompted us to investigate the role of NPM1 on the activation of p300 (autoacetylation). Autoacetylation reaction of p300 was performed in the presence of NPM1 and ³H-acetyl-CoA. NPM1 was found to activate the autoacetylation of p300 in a dose dependent manner **(****Fig. 3f****).** GAPDH was used as a positive control as suggested earlier³⁷. Since, NO signaling induced the p300-autoacetylation, the role of NO on H3K14 acetylation in KB cells was investigated next. GSNO treatment of KB cells enhanced the level of H3K14 acetylation in concentration dependent manner **(****Fig. 3g****),** which was similar to the concomitant increase in NPM1 and GAPDH levels as shown above **(****Fig. 3b****).** Taken together, these data suggest that hyperacetylation of histones in oral cancer could be achieved by overexpressed and autoacetylated-p300, in a NO dependent manner.

### Example 3

### CTK7A is a HAT inhibitor:

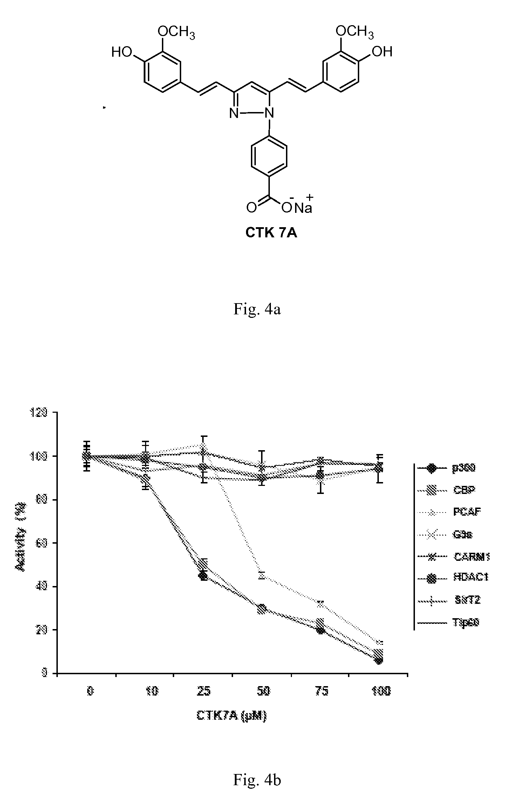

The above mentioned results clearly demonstrate that hyper-activity of lysine acetyltransferase p300, could be one of the factors, responsible for oral cancer manifestation. Therefore, the inhibitor of p300 HAT activity would be useful to verify the possible involvement of the acetyltransferase(s). Using curcumin as synthon, a water soluble derivative, CTK7A was synthesized for this purpose **(****Fig. 4a****).** CTK7A was found to inhibit HAT p300/CBP and PCAF but the activity of other histone modifying enzymes like G9a, CARM1, Tip60, HDACl and SIRT2 were remained unaffected even at 100 µM concentration **(****Fig. 4b****).** Further kinetic analysis revealed that CTK7A follows non-competitive type of inhibition pattern for both the substrate, acetyl-coA and core histone when tested for p300 **(****Fig. 4c****).** However, as expected, CTK7A could efficiently, inhibit the autoacetylation of p300 **(Fig. 4d)** and PCAF **(Fig. 4e)** in vitro, in a concentration dependent manner. Furthermore, CTK7A could also inhibit the enhanced autoacetylation of p300, mediated by cocktail of HDAC inhibitors **(****Fig. 4f****).** To elucidate the effect of CTK7A on the inhibition of histone acetylation in cellular system, KB cells were treated with CTK7A and as expected it could potently inhibit the histone acetylation in KB cells, as potently as the parent compound curcumin³¹ **(****Fig. 4g****).** Taken together, these results suggest that the water soluble HAT inhibitor, CTK7A inhibit histone acetylation in the cellular system, through the inhibition of p300 autoacetylation.

### Example 4

### CTK7A inhibits cell proliferation and induces senescence like growth arrest:

Since p300/CBP is a master regulator and is involved in the regulation of cell cycle progression proliferation, differentiation and in maintaining tissue homeostasis⁴⁵, the growth inhibitory properties of CTK7A in cells were looked into next. KB cells were treated with CTK7A and assayed for the growth inhibitory properties. CTK7A caused a dose dependent inhibition of proliferation of KB cell as assayed by thymidine-incorporation assay **(****Fig. 5a****).** Doxorubicin was used as a positive control **(****Fig. 5a****).**

Antiproliferative activity of CTK7A was further assessed by wound healing assay. It was observed that CTK7A treated cells showed a reduction in the wound healing activity, in the presence of serum **(Fig. 5b).** Cells with or without serum were used as positive and negative controls, respectively **(Fig. 5b).** These results suggest the antiproliferative role of CTK7A in KB cells. Based on the above results, the effect of CTK7A on cell cycle of KB cells was also determined. KB cells were treated with the increased concentration of CTK7A **(****Fig. 5c****)** which caused a dramatic increase in the percentage of polyploid cells (>4N) in a concentration dependent manner **(****Fig. 5c****).**

Induction of polyploidy is often associated with senescence which is known to be associated with antitumor process⁴⁶. It was found that CTK7A treated cells induced the expression of senescence associated beta-galactosidase (SA-β-gal) which is a marker for senescence **(****Fig. 5d****).** This is consistent to the earlier report where the role of p300 in regulating proliferation and senescence of human melanocytes were shown using the p300 specific HATi, lysyl-CoA (a substrate analog of acetyl-CoA). Lysyl-CoA inhibited the proliferation and induced senescence-like growth arrest with expression SA-β-gal⁴⁷.

Cyclin E is a critical regulator of senescence because under overexpression condition it is sufficient to escape from BRG1 and RAS- induced senescence⁴⁸⁻⁴⁹. Given that p300/CBP regulate expression of many cell cycle genes, next the effect of CTK7A on the expression of cyclin E was determined. It was observed that CTK7A down regulated cyclin E expression in a dose dependent manner in KB cells whereas cyclin D1 was affected marginally **(****Fig. 5e****).** Earlier work suggests that cyclin E promoter in human tumor cells and in mouse embryonic fibroblast is regulated by reversible acetylation and deacetylation cycles⁵⁰⁻⁵². Hence, to address the effect of CTK7A on acetylation status at the cyclin E promoter, chromatin immunoprecipitation (ChIP) assays were performed. ChIP assays clearly demonstrated that CTK7A inhibited the acetylation of H3 at the cyclin E promoter **(****Fig. 5f****)**. Taken together, these results indicate that cyclin E down regulation could be a direct cause of p300 HAT inhibitory activity of CTK7A which would be responsible for the senescence-like growth arrest.

### Example 5

### CTK7A inhibits tumor growth:

The in vitro and cell line based studies prompted us to study the role of CTK7A in xenograft mice. It was found that CTK7A is not toxic to the mice after intraperitoneal (i.p) administration. There was no observed weight loss at doses up to 100 mg/kg body weight/ twice a day during 1 month. In order to test the effect of CTK7A on tumor growth, KB cells (2x 10⁶ cells) were inoculated in nude mice in the right and left flanks, respectively and treated them intraperitoneally with 100 mg/kg body weight/ twice a day (for detail see Materials and Methods). CTK7A showed a strong antitumor activity **(****Fig. 6a****).** KB cell tumors were 50% smaller in mice treated with CTK7A than in control untreated mice. The differences in the tumor size between xenografts (Control vs treated) was statistically significant (p < 0.05). The levels of H3 acetylation and that of other proteins (e.g. NPM1, GAPDH, p300) were determined using IHC. CTK7A treated mice tumors showed marginal affect on p300 expression **(****Fig. 6b****).** It was observed that CTK7A decreased the levels of H3K9, K14 acetylation (as probed by H3AcK9AcK14) and ac-p300 **(****Fig. 6b** **and** **Fig. 6c****).** Further to see the site specific effect of CTK7A mediated HAT inhibition on histone acetylation, IHC was done with site specific antibodies. It was found that CTK7A inhibited the H3K14 acetylation more potently than H3K9 acetylation **(****Fig. 6b****),** suggesting that CTK7A mediates inhibition of histone acetylation mainly via inhibition of p300 autoacetylation in mice cancer tissue which could lead to tumor growth inhibition. These data are also consistent with the antiproliferative effect of CTK7A on KB cells **(****Fig. 5a****).** Further, the levels of GAPDH, NPM1 and iNOS were also found to be down regulated in CTK7A treated mice which could be because of antiproliferative affect of CTK7A on mice tumors **(****Fig. 6b****).** Moreover, the levels of COX2, which is induced in many cell types by mitogens, growth factors, cytokine and tumor promoters and has been implicated in many cancers, including oral cancer was also found to be suppressed in CTK7A treated tumors **(****Fig. 6b****).** The down regulation of the proliferation marker was also observed; Ki-67 in CTK7A treated tumors **(****Fig. 6c****)** which support the antiproliferative nature of HATi, CTK7A. It should be noted here that in the control mice, the presence of nuclear GAPDH was observed while nuclear GAPDH was absent or very faintly present in the CTK7A treated mice **(****Fig. 6b****).** These results suggest that CTK7A inhibited the tumor growth in nude mice though its ability to inhibit p300-mediated acetylation.

Although reversible acetylation of histone and nonhistone proteins is one of the most well studied epigenetic marks, the regulation of enzymatic machinery involved in these phenomenons has only recently gained attention. Dysfunction of HDACs and the consequent hypoacetylation of histone as well as nonhistone proteins have been causally related to cancer manifestation for a few cases⁵³. Reports regarding dysfunction of HATs and cancer are rather scanty. Here, it is shown that in oral squamous cell carcinoma (OSCC), histone H3 (predominantly at K14) is hyperacetylated in grade II oral tumors. Associated overexpression and hyperacetylation of histone acetyltransferase p300 suggest that autoacetylation of p300 could be one of the key factors responsible for the histone hyperacetylation. It was found that the histone chaperone NPM1, which is also overexpressed in the oral tumors induce the autoacetylation of p300, presumably through its protein chaperone activity⁵⁴.

These results attribute a new function of the multifunctional nucleolar protein, NPM1.

Furthermore, these studies also establishes an epigenetic signal, IFNγ dependent NO synthesis which could be acting as a basic stimuli for the NPM1 and GAPDH mediated enhanced autoacetylation of p300 and thereby the down stream effect.

The correlation of histone modifications (especially acetylation) and cancer manifestation is not yet well understood. Since cancer is a diverse disease with multiple origins, it does not follow unified cellular rules. However, in case of prostate cancer, it has been shown that acetylation of H3K9, K18, H4K12, and dimethylation of H4R3 are associated with the tumor recurrence⁷. However, the histone modifying enzymes involved in these altered modifications are not yet known. In other words, the molecular mechanisms behind the altered acetylation and methylation are yet to be elucidated. Histones were found to be hyperacetylated in hepatocarcinoma at H3K9 and H4K8²⁴. Significantly, in this case also the enzymatic machinery involved in the hyperacetylation was not studied in detail. Recently, increased acetylation of H3K56, a target of CBP/p300 was observed in multiple cancers⁸. It was also demonstrated that the hyperacetylation of histone, histone chaperone (NPM1) and the metabolic protein, GAPDH is caused by the hyperactive, autoacetylated p300. The unique positive loop involved in this process of hyperacetylation could be a common mechanism in cancer manifestation.

Oral cancer is an inflammation related disease which can lead to NO production that can have an impact on initiation and progression stages of cancer⁴⁴. The present disclosure provide a link between NO production and overexpression of cell proliferative marker genes like NPM1 and GAPDH. These observations is consistent with the report where enhanced iNOS activity has been detected in human tumor cell lines ^{55,56} and in patient tumor samples of various histogenetic origins⁴⁴. NOS activity has also found to be associated with tumorogenesis, proliferation and expression of important signaling components linked to cancer development⁴⁴. It is known that interferon γ (IFNγ), a proinflamatory cytokine activate iNOS to produce NO⁵⁷. It was found that indeed IFNγ treatment to the oral cancer cell line (KB cells) induce the NO production, inhibition of which (IFNγ) represses acetylation of NPM1 and GAPDH.

The results of the present disclosure show that the IFNγ treatment could enhance the nuclear translocation of GAPDH. Although similar nuclear localization of GAPDH has been reported in other case also^{37,58}, the molecular mechanisms of induced nuclear translocation of GAPDH in KB cells remain elusive. Overexpressed NPM1 and GAPDH (nuclear) enhanced the autoacetylation of p300 which in turn induce histone hyperacetylation. The hyperacetylated histones and NPM1 favor the expression of genes responsible for the oral cancer progression **(****Fig.7****)**. NPMland GAPDH induced autoacetylation of p300 can also be used to form more dynamic transcriptional preinitiation complex formation⁵⁹ which result in more efficient transcription. The histone chaperone activity of NPM1 may also be used for transcriptional activation as reported earlier⁶⁰. Cancer cells have higher energy requirement and increased ribosome biosynthesis. Increased levels of NPM1 can also help in the ribosome biogenesis in oral cancer.

Taken together, data of the present disclosure suggest that the acetyltranferase activity of p300 could be one of the factors responsible for the oral cancer. Therefore, the HAT activity could serve as a target for the new generation therapeutics^{15,29}. Here, it was shown that indeed the p300/CBP HAT inhibitor, CTK7A, a water soluble, small molecule compound, derived from curcumin could efficiently inhibit the oral tumor growth in nude mice. The results of the instant disclosure show that induction of polyploidy by the HAT inhibitor (CTK7A) in cells has antitumor activity which induces senescence like growth arrest. Induction of senescence is known to contribute to the treatment of chemotherapy, ionization radiation and is also shown to contribute to the antitumor efficacy of HDAC inhibitors⁶¹.

Current observations establish the casual relationship of hyperacetylation and oral cancer manifestation. Most significantly it elucidates the mechanisms of hyperacetylation and identifies a new candidate protein, NPM1 as a regulator of the master acetyltransferase, p300. With the recent discoveries of several new HAT inhibitors it is being realized that these molecules could be highly useful as therapeutic targets^{15,29}. In oral cancer p300 aceyltransferase activity could be one of the important targets for such molecules, as it was demonstrated by employing CTK7A. However, the complete epigenetic language, which derives OSCC, remains elusive. Small molecule modulators of HATs and HMTases (histone methyltranferases; specially arginine MTase) could be useful to elucidate this epigenetic alteration further and in designing therapeutics.

### References:

1. Parkin DM, Bray F, Ferlay J, Disani P (2005) Global cancer statistics 2002. CA Cancer J Clin 55: 74-108
2. Mao L, Hong WK, Papadimitrakopoulou VA. Focus on head and neck cancer.Cancer Cell. 2004 Apr;5(4):311-6.
3. Quina AS, Buschbeck M, Di Croce L. Chromatin structure and epigenetics. Biochem Pharmacol. 2006 Nov 30;72(11):1563-9.
4. Thorne JL, Campbell MJ, Turner BM. Transcription factors, chromatin and cancer. Int J Biochem Cell Biol. 2009 Jan;41(1):164-75.
5. Fraga, M. F. et al. Loss of acetylation at Lys16 and trimethylation at Lys20 of histone H4 is a common hallmark of human cancer. Nature Genet. 37, 391-400 (2005).
6. Varambally S, Dhanasekaran SM, Zhou M, Barrette TR, Kumar-Sinha C, Sanda MG, Ghosh D, Pienta KJ, Sewalt RG, Otte AP, Rubin MA, Chinnaiyan AM. The polycomb group protein EZH2 is involved in progression of prostate cancer. Nature. 2002 Oct 10;419(6907):624-9.
7. Seligson DB, Horvath S, Shi T, et al. Global histone modification patterns predict risk of prostate cancer recurrence. Nature 2005 ; 435 (7046): 1262 -6.
8. Das C, Lucia MS, Hansen KC, Tyler JK.CBP/p300-mediated acetylation of histone H3 on lysine 56. Nature. 2009;459(7243):113-7.
9. Pfister S, Rea S, Taipale M, Mendrzyk F, Straub B, Ittrich C, Thuerigen O, Sinn HP, Akhtar A, Lichter P. The histone acetyltransferase hMOF is frequently downregulated in primary breast carcinoma and medulloblastoma and constitutes a biomarker for clinical outcome in medulloblastoma. Int J Cancer. 2008 ;122(6):1207-13.
10. Esteller M. Cancer epigenomics: DNA methylomes and histone-modification maps. Nat Rev Genet. 2007, 8(4):286-98.
11. Eberharter A, Becker PB. Histone acetylation: a switch between repressive and permissive chromatin. Second in review series on chromatin dynamics. EMBO Rep. 2002 ;3(3):224-9.
12. Carrozza MJ, Utley RT, Workman JL, Cote J. The diverse functions of histone acetyltransferase complexes. Trends Genet. 2003 ;19(6):321-9. Review.
13. Berger, S. L. Histone modifications in transcriptional regulation. Curr. Opin. Genet. Dev. 2002, 12, 142-148.
14. Bhaumik SR, Smith E, Shilatifard A. Covalent modifications of histones during development and disease pathogenesis. Nat Struct Mol Biol. 2007;14(11):1008-2007
15. Selvi RB, Kundu TK. Reversible acetylation of chromatin: implication in regulation of gene expression, disease and therapeutics. Biotechnol J. 2009 Mar;4(3):375-90.
16. Das C, Kundu TK. Transcriptional regulation by the acetylation of nonhistone proteins in humans - a new target for therapeutics. IUBMB Life. 2005 Mar;57(3):137-49.
17. Spange S, Wagner T, Heinzel T, Krämer OH. Acetylation of non-histone proteins modulates cellular signalling at multiple levels. Int J Biochem Cell Biol. 2009 Jan;41(1):185-98.
18. Glozak MA, Seto E. Histone deacetylases and cancer. Oncogene. 2007 Aug 13;26(37):5420-32.
19. Van Beekum O, Kalkhoven E. Aberrant forms of histone acetyltransferases in human disease. Subcell Biochem. 2007;41:233-62.
20. Shikama, N.; Ivon, J.; La Thangue, N. The p300/CBP family: Integrating signals with transcription factors and chromatin. Trends Cell Biol.1997, 7, 230-236.
21. Debes JD, Sebo TJ, Lohse CM, Murphy LM, Haugen DA, Tindall DJ. p300 in prostate cancer proliferation and progression. Cancer Res. 2003 Nov15;63(22):7638-40.
22. Ishihama K, Yamakawa M, Semba S, Takeda H, Kawata S, Kimura S, Kimura W. Expression of HDACl and CBP/p300 in human colorectal carcinomas. J Clin Pathol. 2007 Nov;60(11):1205-10.
23. Gayther SA, Batley SJ, Linger L, et al. Mutations truncating the EP300 acetylase in human cancers. Nat Genet 2000 ; 24 (3): 300 -3.
24. Bai X, Wu L, Liang T, Liu Z, Li J, Li D, Xie H, Yin S, Yu J, Lin Q, Zheng S. Overexpression of myocyte enhancer factor 2 and histone hyperacetylation in hepatocellular carcinoma. J Cancer Res Clin Oncol. 2008 Jan;134(1):83-91.
25. Varier, R. A.; Kundu, T. K. Chromatin modifications (acetylation/ deacetylation/ methylation) as new targets for HIV therapy. Current Pharmaceutical Design 2006, 12, 1975-1993
26. Davidson SM, Townsend PA, Carroll C, Yurek-George A, Balasubramanyam K, Kundu TK, Stephanou A, Packham G, Ganesan A, Latchman DS. The transcriptional coactivator p300 plays a critical role in the hypertrophic and protective pathways induced by phenylephrine in cardiac cells but is specific to the hypertrophic effect of urocortin. Chembiochem. 2005 Jan;6(1):162-70.
27. Zhou, X. Y.; Shibusawa, N.; Naik, K.; Porras, D.; Temple, K.; Ou, H.; Kaihara, K.; Roe, M. W.; Brady, M. J.; Wondisford, F. E. Insulin regulation of hepatic gluconeogenesis through phosphorylation of CREB-binding protein. Nat. Med. 2004, 10,633-637.
28. Rouaux, C.; Jokic, N.; Mbebi, C.; Boutillier, S.; Loeffler, J. P.; Boutillier, A. L. Critical loss of CBP/p300 histone acetylase activity by caspase-6 during neurodegeneration. EMBO J. 2003, 22, 6537-6549.
29. Cole PA. Chemical probes for histone-modifying enzymes. Nat Chem Biol. 2008 Oct;4(10):590-7.
30. Mantelingu K, Reddy BA, Swaminathan V, Kishore AH, Siddappa NB, Kumar GV, Nagashankar G, Natesh N, Roy S, Sadhale PP, Ranga U, Narayana C, Kundu TK. Specific inhibition of p300-HAT alters global gene expression and represses HIV replication. Chem Biol. 2007 Jun;14(6):645-57.
31. Balasubramanyam K, Varier RA, Altaf M, et al. Curcumin, a novel p300/CREB-binding protein-specific inhibitor of acetyltransferase, represses the acetylation of histone/nonhistone proteins and histone acetyltransferase-dependent chromatin transcription. J Biol Chem 2004 ; 279 (49): 51163 -71
32. Morimoto, T. et al. The dietary compound curcumin inhibits p300 histone acetyltransferase activity and prevents heart failure in rats. J. Clin. Invest. 118, 868-878 (2008).
33. Grisendi S, Mecucci C, Falini B, Pandolfi PP. Nucleophosmin and cancer. Nat Rev Cancer. 2006 Jul;6(7):493-505.
34. Altenberg, B. & Greulich, K. O. Genes of glycolysis are ubiquitously overexpressed in 24 cancer classes. Genomics 84, 1014-1020 (2004).
35. Majumder, P. K. et al. mTOR inhibition reverses Akt-dependent prostate intraepithelial neoplasia through regulation of apoptotic and HIF-1-dependent pathways. Nature Med. 10, 594-601 (2004).
36. Thompson, P. R.; Wang, D.; Wang, L.; Fulco, M.; Pediconi, N.; Zhang, D.; An, W.; Ge, Q.; Roeder, R. G.; Wong, J.; Levrero, M.; Sartorelli, V.; Cotter, R. J.; Cole, P. A. Regulation of the p300 HAT domain via a novel activation loop. Nat. Struct. Mol. Biol. 2004, 11, 308-315.
37. Sen N, Hara MR, Kornberg MD, Cascio MB, Bae BI, Shahani N, Thomas B, Dawson TM, Dawson VL, Snyder SH, Sawa A. Nitric oxide-induced nuclear GAPDH activates p300/CBP and mediates apoptosis. Nat Cell Biol. 2008 Jul;10(7):866-73.
38. Hansson ML, Popko-Scibor AE, Saint Just Ribeiro M, Dancy BM, Lindberg MJ, Cole PA, Wallberg AE. The transcriptional coactivator MAML1 regulates p300 autoacetylation and HAT activity. Nucleic Acids Res. 2009 May;37(9):2996-3006.
39. Yang XJ, Seto E. Lysine acetylation: codified crosstalk with other posttranslational modifications. Mol Cell. 2008 Aug 22;31(4):449-61. Review
40. Huang WC, Chen CC. Akt phosphorylation of p300 at Ser-1834 is essential for its histone acetyltransferase and transcriptional activity. Mol Cell Biol. 2005 Aug;25(15):6592-602
41. Moncada, S., Palmer, R. M. & Higgs, E. A. Nitric oxide: physiology, pathophysiology and pharmacology. Pharmacol. Rev. 43, 109-142 (1991).
42. Gallo O, Masini E, Morbidelli L, Franchi A, Fini-Storchi I, Vergari WA, Ziche M. Role of nitric oxide in angiogenesis and tumor progression in head and neck cancer. J Natl Cancer Inst. 1998 Apr 15;90(8):587-96
43. Czesnikiewicz-Guzik M, Lorkowska B, Zapala J, Czajka M, Szuta M, Loster B, Guzik TJ, Korbut R. NADPH oxidase and uncoupled nitric oxide synthase are major sources of reactive oxygen species in oral squamous cell carcinoma. Potential implications for immune regulation in high oxidative stress conditions. J Physiol Pharmacol. 2008 Mar;59(1):139-52
44. Fukumura D, Kashiwagi S, Jain RK. The role of nitric oxide in tumour progression. Nat Rev Cancer. 2006 Jul;6(7):521-34. Review
45.Goodman, R. H.; Smolik, S. CBP/p300 in cell growth, transformation, and development. Genes Dev. 2000, 14, 1553-1577.
46. Ota H, Tokunaga E, Chang K, Hikasa M, Iijima K, Eto M, Kozaki K, Akishita M, Ouchi Y, Kaneki M. Sirt1 inhibitor, Sirtinol, induces senescence-like growth arrest with attenuated Ras-MAPK signaling in human cancer cells. Oncogene. 2006 Jan 12;25(2):176-85.
47. Bandyopadhyay D, Okan NA, Bales E, Nascimento L, Cole PA, Medrano EE. Down-regulation of p300/CBP histone acetyltransferase activates a senescence checkpoint in human melanocytes. Cancer Res. 2002 Nov 1;62(21):6231-9.
48. Peeper, D. S., Shvarts, A., Brummelkamp, T., Douma, S., Koh, E. Y., Daley, G. Q., and Bernards, R. A functional screen identifies hDRIL1 as an oncogene that rescues RAS-induced senescence. Nat. Cell. Biol., 4: 148-153, 2002.
49. Shanahan, F., Seghezzi, W., Parry, D., Mahony, D., and Lees, E. Cyclin E associates with BAF155 and BRG1, components of the mammalian SWI-SNF complex, and alters the ability of BRG1 to induce growth arrest. Mol. Cell. Biol., 19: 1460-1469, 1999.
50. Brehm, A., Miska, E. A., McCance, D. J., Reid, J. L., Bannister, A. J., and Kouzarides T. Retinoblastoma protein recruits histone deacetylase to repress transcription. Nature (Lond.), 391: 597-600, 1998.
51. Sambucetti, L. C., Fischer, Denise D., Zabludoff, S, Kwon, P. O., Chamberlin, H, Trogani, N, Xu, H., and Cohen, D. Histone deacetylase inhibition selectively alters the activity and expression of cell cycle proteins leading to specific chromatin acetylation and antiproliferative effects. J. Biol. Chem., 274: 34940-34947, 1999.
52. Morrison, A. J., Sardet, C., and Herrera, R. E. Retinoblastoma protein transcriptional repression through histone deacetylation of a single nucleosome. Mol. Cell. Biol., 22: 856-865, 2002.
53. Bolden JE, Peart MJ, Johnstone RW. Anticancer activities of histone deacetylase inhibitors. Nat Rev Drug Discov. 2006 Sep;5(9):769-84. Review.
54. Szebeni A, Olson MO. Nucleolar protein B23 has molecular chaperone activities. Protein Sci. 1999 Apr;8(4):905-12.
55. Jenkins, D. C. et al. Human colon cancer cell lines show a diverse pattern of nitric oxide synthase gene expression and nitric oxide generation. Brit. J. Cancer 70, 847-849 (1994).
56. Asano, K. et al. Constitutive and inducible nitric oxide synthase gene expression, regulation, and activity in human lung epithelial cells. Proc. Natl Acad. Sci. USA 91, 10089-10093 (1994).
57. Wu, K. K. 1995. Inducible cyclooxygenase and nitric oxide synthase. Adv. Pharmacol. 33:179*.*
58. Hara MR, Agrawal N, Kim SF, Cascio MB, Fujimuro M, Ozeki Y, Takahashi M, Cheah JH, Tankou SK, Hester LD, Ferris CD, Hayward SD, Snyder SH, Sawa A. S-nitrosylated GAPDH initiates apoptotic cell death by nuclear translocation following Siah1 binding. Nat Cell Biol. 2005 Jul;7(7):665-74.
59. Black, J. C.; Choi, J. E.; Lombardo, S. R.; Carey, M. A Mechanism for Coordinating Chromatin Modification and Preinitiation Complex Assembly. Mol. Cell 2006, 23, 809-818.
60. Swaminathan V, Kishore AH, Febitha KK, Kundu TK. Human histone chaperone nucleophosmin enhances acetylation-dependent chromatin transcription. Mol Cell Biol. 2005 Sep;25(17):7534-45.
61. Dimri GP. What has senescence got to do with cancer? Cancer Cell. 2005 Jun;7(6):505-12. Review.

## Claims

1. An *in vitro* method of inhibiting histone acetyltransferases (HATs) by Sodium-4-(3,5-bis(4-hydroxy-3-methoxystyryl)-1*H*-pyrazol-1-yl)benzoate (CTK7A), said method comprising step of incubating the HATs with CTK7A,
wherein the HATs are CREB binding protein (p300/CBP) and P300/CBP Associated factor (PCAF).

2. The method as claimed in claim 1, wherein HAT inhibitory concentration of the CTK7A ranges from 25µM to 200 µM, preferably 40 µM to 80 µM.

3. A therapeutically acceptable amount of Sodium-4-(3,5-bis(4-hydroxy-3-methoxystyryl)-1H-pyrazol-1-yl)benzoate (CTK7A) for use in treating cancer, optionally along with pharmaceutically acceptable excipients, wherein the CTK7A inhibits acetyltransferase activity of HATs and thereby inhibits hyperacetylation of histones.

4. The therapeutically acceptable amount of CTK7A for use according to claim 3, wherein CTK7A is to be administered intraperitonially.

5. The therapeutically acceptable amount of CTK7A for use according to claim 3, wherein the CTK7A reduces tumor size of the cancer by 50%.

6. The therapeutically acceptable amount of CTK7A for use according to claim 3, wherein the CTK7A induces polyploidy in cancer cells to induce senescence like growth arrest.

7. The therapeutically acceptable amount of CTK7A for use according to claim 3, wherein the cancer is oral squamous cell carcinoma.

8. The therapeutically acceptable amount of CTK7A for use according to claim 3, wherein the CTK7A is further administered along with an epigenetic drug target molecule or pharmaceutically acceptable chemotherapeutic or a combination thereof.

9. An *in vitro* method of inhibiting autoacetylation of p300 by Sodium-4-(3,5-bis(4-hydroxy-3-methoxystyryl)-1*H*-pyrazol-1-yl)benzoate (CTK7A), said method comprising steps of:
reacting and incubating full length radio-labeled p300 with predetermined concentrations of CTK7A, optionally along with cocktail of HDAC inhibitors, and
performing filter binding assay and identifying inhibition of autoacetylation of p300 by fluorography and autoradiography.

10. Sodium-4-(3,5-bis(4-hydroxy-3-methoxystyryl)-1*H*-pyrazol-1-yl)benzoate (CTK7A).

11. A process for preparing Sodium-4-(3,5-bis(4-hydroxy-3-methoxystyryl)-1*H*-pyrazol-1-yl)benzoate (CTK7A), said process comprising the step of reacting hydrazinobenzoylcurcumin with sodium ethoxide to obtain Sodium-4-(3,5-bis(4-hydroxy-3-methoxystyryl)-1H-pyrazol-1-yl)benzoate (CTK7A).

12. Use of Sodium-4-(3,5-bis(4-hydroxy-3-methoxystyryl)-1*H*-pyrazol-1-yl)benzoate (CTK7A), optionally along with pharmaceutically acceptable excipients, for the preparation of a medicament for treating cancer, wherein the CTK7A inhibits acetyltransferase activity of HATs and thereby inhibits hyperacetylation of histones.

13. The use according to claim 12, wherein CTK7A is to be administered intraperitonially.

14. The use according to claim 12, wherein the cancer is oral squamous cell carcinoma.

15. The use according to claim 12, wherein the CTK7A is to be further administered along with an epigenetic drug target molecule or pharmaceutically acceptable chemotherapeutic or a combination thereof.

## Patentansprüche

1. Ein in vitro Verfahren zur Hemmung von Histonacetyltransferasen (HATs) mit Natrium-4-(3,5-bis (4-hydroxy-3-methoxystyryl)-1H-pyrazol-1-yl)benzoat (CTK7A), wobei das Verfahren einen Schritt umfasst indem die HATs mit CTK7A inkubiert werden, wobei die HATs CREB-Bindeprotein (p300/CBP) und P300/CBP assoziierter Faktor (PCAF) sind.

2. das Verfahren gemäß Anspruch 1, wobei die HRT Helmkonzentration von CTK7A von 25 µM bis 200 µM reicht, vorzugsweise von 40 µM bis 80 µM.

3. Eine therapeutisch akzeptierbare Menge von Natrium-4-(3,5-bis (4-hydroxy-3-methoxystyryl)-1H-pyrazol-1-yl)benzoat (CTK7A) zur Verwendung in der Behandlung von Krebs, wahlweise zusammen mit pharmazeutisch akzeptierbaren Hilfsstoffen, wobei CTK7A die Acetyltransferraseaktivität von HATs hemmt und dadurch die Hyperacetylierung von Histonen hemmt.

4. Die therapeutisch akzeptierbare Menge von CTK7A zur Verwendung gemäß Anspruch 3, wobei CTK7A intraperitoneal verabreicht wird.

5. Die therapeutisch akzeptierbare Menge von CTK7A zur Verwendung gemäß Anspruch 3, wobei CTK7A die Tumorgröße des Krebses um 50 % reduziert.

6. Die therapeutisch akzeptierbare Menge von CTK7A zur Verwendung gemäß Anspruch 3, wobei CTK7A Polyploidie in Krebszellen induziert, um einen Seneszenz-ähnlichen Wachstumsarrest zu induzieren.

7. Die therapeutisch akzeptierbare Menge von CTK7A zur Verwendung gemäß Anspruch 3, wobei der Krebs ein orales Plattenepithelkarzinom ist.

8. Die therapeutisch akzeptierbare Menge von CTK7A zur Verwendung gemäß Anspruch 3, wobei CTK7A zusammen mit einem epigenetischen Arzneimittelzielmolekül, oder pharmazeutisch akzeptierbaren Chemotherapeutikum, oder einer Kombination davon verabreicht wird.

9. Ein in vitro Verfahren zur Hemmung der Autoacetylierung von p300 durch Natrium-4-(3,5-bis (4-hydroxy-3-methoxystyryl)-1H-pyrazol-1-yl)benzoat (CTK7A), wobei das Verfahren die folgenden Schritte umfasst: Reagieren von und Inkubieren von radioaktiv markiertem Volllängen-p300 mit einer vorbestimmten Konzentration von CTK7A, wahlweise zusammen mit einer Mischung von HDAC Inhibitoren; und Durchführen eines Filterbindungstests und Identifizieren der Hemmung der Autoacetylierung von p300 mittels Fluorographie oder Autoradiographie.

10. Natrium-4-(3,5-bis (4-hydroxy-3-methoxystyryl)-1H-pyrazol-1-yl)benzoat (CTK7A).

11. Ein Verfahren zur Herstellung von Natrium-4-(3,5-bis (4-hydroxy-3-methoxystyryl)-1H-pyrazol-1-yl)benzoat (CTK7A), wobei das Verfahren den Schritt der Reaktion von Hydrazinobenzoylcurcumin mit Natriumethanolat umfasst, um Natrium-4-(3,5-bis (4-hydroxy-3-methoxystyryl)-1H-pyrazol-1-yl)benzoat (CTK7A) zu erhalten.

12. Verwendung von von Natrium-4-(3,5-bis (4-hydroxy-3-methoxystyryl)-1H-pyrazol-1-yl)benzoat (CTK7A), wahlweise zusammen mit pharmazeutisch akzeptierbar Hilfsstoffen, zur Herstellung eines Arzneimittels zur Behandlung von Krebs, wobei CTK7A die Acetyltransferaseaktivität der HATs hemmt und dadurch die Hyperacetylierung von Histonen hemmt.

13. die Verwendung gemäß Anspruch 12, wobei CTK7A intraperitoneal verabreicht wird.

14. die Verwendung gemäß Anspruch 12, wobei der Krebs ein orales Plattenepithelkarzinom ist.

15. die Verwendung gemäß Anspruch 12, wobei CTK7A zusammen mit einem epigenetischen Arzneimittelzielmolekül, oder pharmazeutisch akzeptierbaren Chemotherapeutikum, oder einer Kombination davon verabreicht wird.

## Revendications

1. Procédé *in vitro* d'inhibition d'histone acétyltransférases (HAT) par le sodium-4-(3,5-bis(4-hydroxy-3-méthoxystyryl)-1*H*-pyrazol-1-yl)benzoate (CTK7A), ledit procédé comprenant l'étape d'incubation des HAT avec CTK7A,
où les HAT sont la protéine de liaison CREB (p300/CBP) et le facteur associé à P300/CBP (PCAF).

2. Procédé tel que revendiqué selon la revendication 1, où la concentration inhibitrice HAT de CTK7A s'étend de 25 µM à 200 µM, préférablement de 40 µM à 80 µM.

3. Quantité thérapeutiquement acceptable de sodium-4-(3,5-bis(4-hydroxy-3-méthoxystyryl)-1*H*-pyrazol-1-yl)benzoate (CTK7A) à utiliser dans le traitement du cancer, éventuellement conjointement à des excipients pharmaceutiquement acceptables, où le CTK7A inhibe l'activité acétyltransférase des HAT et inhibe de là l'hyperacétylation des histones.

4. Quantité thérapeutiquement acceptable de CTK7A pour l'utilisation selon la revendication 3, où le CTK7A doit être administré par voie intrapéritonéale.

5. Quantité thérapeutiquement acceptable de CTK7A pour l'utilisation selon la revendication 3, où le CTK7A réduit la taille des tumeurs du cancer de 50 %.

6. Quantité thérapeutiquement acceptable de CTK7A pour l'utilisation selon la revendication 3, où le CTK7A induit la polyploïdie dans les cellules cancéreuses induisant la sénescence telle que l'arrêt de croissance.

7. Quantité thérapeutiquement acceptable de CTK7A pour l'utilisation selon la revendication 3, où le cancer est le carcinome des cellules squameuses buccales.

8. Quantité thérapeutiquement acceptable de CTK7A pour l'utilisation selon la revendication 3, où le CTK7A est en outre administré conjointement à une molécule cible épigénétique de médicament ou un agent chimiothérapeutique ou une combinaison pharmaceutiquement acceptable de celui-ci.

9. Procédé *in vitro* d'inhibition de l'autoacétylation de p300 par le sodium-4-(3,5-bis(4-hydroxy-3-méthoxystyryl)-1*H*-pyrazol-1-yl)benzoate (CTK7A), ledit procédé comprenant les étapes de :
réaction et d'incubation de p300 radiomarqué de pleine longueur avec des concentrations prédéterminées de CTK7A, éventuellement conjointement à un cocktail d'inhibiteurs HDAC, et
l'exécution de l'essai de liaison sur filtre et l'identification de l'inhibition de l'autoacétylation de p300 par fluorographie et autoradiographie.

10. Sodium-4-(3,5-bis(4-hydroxy-3-méthoxystyryl)-1*H*-pyrazol-1-yl)benzoate (CTK7A).

11. Procédé de préparation de sodium-4-(3,5-bis(4-hydroxy-3-méthoxystyryl)-1*H*-pyrazol-1-yl)benzoate (CTK7A), ledit procédé comprenant les étapes de réaction d'hydrazinobenzoylcurcumin avec de l'éthoxyde de sodium pour obtenir du sodium-4-(3,5-bis(4-hydroxy-3-méthoxystyryl)-1*H*-pyrazol-1-yl)benzoate (CTK7A).

12. Utilisation du sodium-4-(3,5-bis(4-hydroxy-3-méthoxystyryl)-1*H*-pyrazol-1-yl)benzoate (CTK7A), conjointement éventuellement à des excipients pharmaceutiquement acceptables, pour la préparation d'un médicament de traitement du cancer, où le CTK7A inhibe l'activité acétyltransférase des HAT et inhibe de là l'hyperacétylation des histones.

13. Utilisation selon la revendication 12, où le CTK7A doit être administré par voie intrapéritonéale.

14. Utilisation selon la revendication 12, où le cancer est le carcinome des cellules squameuses buccales.

15. Utilisation selon la revendication 12, où le CTK7A doit être administré en outre conjointement à une molécule cible médicamenteuse épigénétique ou un agent chimiothérapeutique ou une combinaison pharmaceutiquement acceptable de celui-ci.
